# EUROPEAN PATENT SPECIFICATION

(11) **EP 4 582 125 B1**
(45) Date of publication and mention of the grant of the patent: **11.11.2026**
(21) Application number: 24150428.1
(22) Date of filing: 04.01.2024
(51) Int. Cl.: A61M 16/06, A61M 16/08

(54) **PEDIATRIC NASAL MASK**
PÄDIATRISCHE NASENMASKE
MASQUE NASAL PÉDIATRIQUE

(43) Date of publication of application: 09.07.2025
(73) Proprietor: Air Liquide Medical Systems, 92182 Antony Cedex (FR)
(72) Inventor: ALBERICI, Luca, 25073 Bovezzo (IT); MASSERDOTTI, Fulvio, 25073 Bovezzo (IT); SANDONI, Giuseppe, 25073 Bovezzo (IT)
(74) Representative: Air Liquide

(56) References cited:
- WO-A1-2020/178686
- WO-A1-2022/122974
- US-A1- 2015 328 423
- US-B2- 11 077 272
- US-B2- 11 351 323
- US-B2- 11 497 874

## Description

The invention concerns a pediatric respiratory nasal mask for use in the treatment of respiratory conditions or diseases affecting little infants, i.e. babies, newborns or the like, whatever their facial morphology, preferably infants up to 3 years and/or of from about 3 to 12 kg, i.e. from newborns to 3-year old children.

Nasal respiratory masks are commonly used for delivering a flow of breathable gas for, or to assist in, patient respiration, in particular for providing a non-invasive positive pressure ventilation (NPPV) or a nasal continuous positive airway pressure (N-CPAP) therapy to patients in need thereof.

Some mask assemblies, developed for adults patients, comprise :
- a (semi-)rigid hollow shell forming the front part or mask body;
- a soft face-contacting cushion that is coupled to the hollow shell and comes into contact with and conforms to the various facial contours of the patient face, and
- an upper arm with a forehead support that comes into contact with the forehead of the user, as well as two lateral arms projecting on the left and right sides of the mask body.

The cushion is usually made of soft, resilient elastomeric material, such as soft silicone or similar, whereas the hollow shell is made of a more rigid material, such as plastic material or the like.

A headgear attached to the upper and lateral arms is used for correctly positioning, maintaining and/or securing the mask on the face/head of the patient.

Examples of a various nasal masks are disclosed by EP462701, EP874667, EP197235, DE2114500, WO2008/037031, EP2818194, WO2007/045023 and WO2000/57942.

It is known that nasal respiratory masks designed for adults cannot be used for delivering gases to infants, i.e. children, babies or the like, as their facial morphologies are very different, which involves constraints for well positioning and maintaining the mask on the infant's face, leading to discomfort, gas leaks and other issues.

Pediatric nasal masks have been specially designed for infants. Thus, EP3246065 teaches a light and soft pediatric mask assembly comprising a mask body, a holding arm with a forehead structure, a cushion and two lateral arms that are made of a resilient soft material, such as silicone or the like, preferably made of one piece, by injection-molding or the like.

However, it appears that those developed for infants of 7 years or older, are usually not adapted to younger infants, especially newborns and little children of less than 3 years old, due to very dissimilar facial morphologies.

Further, it has been also noticed, in some cases, gas leaks appearing between the tubular connector, e.g. an elbow connector, that is plugged into the inlet orifice of the mask body, especially when the cushion is made of a resilient soft material, such as silicone, or worse, undesirable unplugging of the tubular connector that is fed with pressurized respiratory gas provided by a flexible hose fluidly connected to a medical ventilator or the like.

Arranging a rigid ring element in the inlet orifice for reinforcing the connection between the tubular connector and the mask body is not fully satisfying, especially when the tubular connector comprises a spherical head for being rotatable, as it leads to leaks and/or disconnection of the tubular connector.

Other masks structures are disclosed by US11077272, US11351323, US11497874, WO2020/178686, WO2022/122974 and US2015/328423.

A goal of the present invention is to provide an improved pediatric nasal mask that is well suited to a little infant, i.e. a 3-year old child or younger, such as a baby, a newborn, a toddler or a little child, when used for treating said infant, i.e. when a gaseous treatment is delivered by means of said nasal mask, thereby ensuring efficient gas tightness (seal) and avoiding or limiting disconnection of the tubular connector that is plugged in the mask body.

The invention is defined in the appended claims. A solution according to the present disclosure concerns a pediatric nasal mask, i.e. a respiratory nasal mask for pediatric uses, comprising:
- a one-piece hollow molded structure made of a resilient soft material, said one-piece hollow molded structure comprising a front body, a rear cushion and an inner chamber, wherein :
   . the front body comprises an inlet orifice opening in the inner chamber, and
   . the cushion comprises a rear aperture, i.e. nose opening, adapted for receiving at least part of the infant's nose, when the infant wears the mask, said rear aperture being in fluid communication with the inner chamber,
- and a tubular element made of a rigid polymer arranged in the inlet orifice of the front body.

Further, in the pediatric mask of the disclosure:
- the tubular element is arranged on a support element, the support element being a plate structure having a curved triangular shape with rounded vertices, and
- the one-piece molded structure is over-molded over said support element.

The pediatric mask according to the present disclosure can further comprise one or more of the following additional features:
- the one-piece hollow molded structure delimits the inner chamber, i.e. an inner compartment containing the gas.
- the tubular element made of a rigid polymer is coaxially (XX) arranged in the inlet orifice of the front body.
- the tubular element has a general cylindrical shape.
- the tubular element comprise an inner passage, i.e. a lumen.
- the tubular element comprise an inner passage with an inlet and an outlet, i.e. orifices located at the opposite ends of the inner passage.
- the plate structure is not planar.
- the support element, in particular the plate structure, has a height of between 25 mm and 35 mm and a base of between 30 mm and 40 mm.
- the support element, in particular the plate structure, has a wall thickness of between 1.5 mm and 3 mm.
- the support element, in particular the plate structure, comprises several traversing holes.
- the tubular element and the support element form or constitute a one-piece assembly, preferably a rigid or semi-rigid assembly.
- the support element and the tubular element (i.e. the one-piece assembly) are made of a rigid polymer, i.e. a plastic material.
- the one-piece assembly comprising the support element and the tubular element is manufactured by injection molding or any suitable manufacturing process.
- the tubular element and/or the support element is made of a polymer chosen among polyamide (PA), polycarbonate (PC) and polybutylene terephthalate (PBT), or any other plastic/polymer material.
- the tubular element protrudes on a first surface of the support element.
- the tubular element has an inner diameter (of its lumen) of between about 12 mm and about 19 mm, preferably of between 14 and 18 mm, more preferably of between 15,5 mm and 18 mm.
- the tubular element has an outer diameter of between about 18 and 21 mm.
- the tubular element has length of between about 6 and 10 mm.
- the support element is traversed by an opening that is fluid communication with the inner passage of the tubular element.
- the one-piece molded structure further comprises a holding arm projecting upwardly, i.e. an upper arm.
- the holding arm is integral with the one-piece molded structure.
- the one-piece molded structure further comprises two lateral arms arranged on each side (i.e. left and right sides) and projecting laterally.
- the front body, the holding arm, the rear cushion and the two lateral arms are molded in one piece.
- the front body, the holding arm, the rear cushion and the two lateral arms are made of the resilient soft material.
- the resilient soft material comprises silicone or the like, preferably silicone.
- the tubular element comprises an inner wall forming a ball-socket structure.
- at least a part of the inner wall of the tubular element has a semi-spherical shape.
- at least a part of the inner wall of the tubular element is configured to lodge a ball-end of a tubular connector, i.e. a (partially) spherical head.
- the pediatric mask comprises a tubular connector comprising a ball-end inserted into the ball-socket structure of the tubular element.
- the ball-end of the tubular connector is rotatably maintained in the ball-socket structure of the tubular element.
- the shape of the ball-end of the tubular connector matches the shape of the inner wall of the tubular element.
- the cushion comprises at least one flexible membrane bordering the rear aperture, preferably a soft flexible membrane.
- the flexible membrane has a thickness of less than 0,6 mm, preferably less than 0,5 mm.
- the flexible membrane has a thickness of more than 0,1 mm, preferably more than 0,2 mm.
- the flexible membrane has a substantially equal or constant thickness around the central aperture, i.e. in the regions of the membrane located above, beneath and on both sides of the nose of the infant, when the mask is donned, i.e. in use.
- the flexible membrane has a width of less than 20 mm, preferably less than 15 mm.
- the one-piece hollow molded structure forming the mask body has a generally triangular tridimensional shape.
- the one-piece hollow molded structure has/comprises a height of at least 80 mm, preferably of between 85 and 90 mm, more preferably of about 87 mm.
- the one-piece hollow molded structure has/comprises a width of less than 90 mm, preferably of between 80 and 85 mm, more preferably of about 83 mm.
- the mask is dimensioned for fitting to the nasal region of an infant under 3 years (i.e. newborn, baby, toddler, little child or the like) and/or weighting less than 12 kg, preferably of between about 3 kg and 12 kg.
- the front body comprises a front panel, i.e. a front wall structure.
- the front panel comprises an outer wall and an inner wall.
- the support element is sandwiched between the outer wall and the inner wall of the front panel.

The pediatric mask according to the present disclosure can further comprise one or more of the following additional features:
- the mask has a weight of less than 50 g, preferably of less than 40 g.
- the mask has a weight of less than 30 g, preferably of between 10 and 20 g.
- one of the two lateral arms projects from the right side of the mask body (i.e. a right lateral arm), while the other of the two lateral arms projects from the left side of the mask body (i.e. a left lateral arm).
- the flexible membrane surrounds the rear aperture, i.e. central aperture, that receives at least part of the nose of the patient, when donned by said patient.
- the membrane is made of said resilient soft material and is integral with the rest of the cushion.
- the holding arm comprises at least one slot at its terminal end, typically several slots, such as 3 slots, for fixing thereto straps of a headgear.
- the two lateral arms projecting laterally comprise (at least) a slot or a similar aperture for fixing thereto straps of a headgear.
- a headgear is attached to the holding arm and the two lateral arms.
- the holding arm projecting upwardly and the two opposite lateral arms projecting laterally attached to corners, vertices or angle regions of the triangular-shaped one-piece hollow molded structure.
- the inlet orifice, i.e. front orifice, is arranged at (about) the center of the one-piece hollow molded structure.
- the two opposite lateral arms have a generally curved-shape, preferably the two lateral arms are curved toward the rear and bottom sides of the mask.
- the mask is pediatric nasal mask dimensioned, sized and/or designed for fitting to the nasal region of an little infant under 3 years and/or weighting less than 12 kg, e.g. a little child, a toddler, a newborn, baby or any similar pediatric patient.
- the flexible membrane of the cushion comprises a peripheral rim delimiting the rear aperture.
- the regions of the flexible membrane comprising the peripheral rim can be flat or slightly incurved toward the inner volume of the cushion, i.e. the inner chamber.
- the peripheral rim of the cushion has a generally-triangular shape or any other shapes allowing receiving the nose regions of the pediatric patient while ensuring gas tightness and good comfort of use.
- the peripheral rim of the cushion has a substantially generally-triangular shape including three side borders forming said triangular shape.
- the side borders of the triangular-shaped rim have a curved shape oriented toward the center of the rear aperture, i.e. the side borders are curved.

The present disclosure further concerns a ventilation assembly comprising a gas delivery device, i.e. an ventilation apparatus that delivers a pressurized respiratory gas, such as air, and a pediatric mask according to the present invention, said gas delivery device being fluidly connected to the pediatric mask by means of a flexible hose, i.e. a gas line, for providing a respiratory gas to the pediatric mask having a pressure of between about 4 and 24 cmH₂0.

The flexible hose comprises a tubular connector comprising a ball-end that is rotatably coupled to the ball-socket structure of the tubular element of the pediatric mask of the present invention.

Furthermore, the present disclosure also concerns a method for providing a pressurized respiratory gas to a pediatric patient under 3 years and/or of less than 12 kg comprising :
a) providing a pressurized respiratory gas at a pressure of between about 4 and 24 cmH₂0, and
b) delivering the pressurized respiratory gas to the airways of the pediatric patient in need thereof by means of a pediatric nasal mask according to the present disclosure.

The method according to the present disclosure can further comprise one or more of the following additional features:
- the pressurized respiratory gas is provided by a ventilation apparatus or the like.
- the pressurized respiratory gas comprises air or an air/oxygen mixture.
- the pediatric patient suffers from a respiratory disorder or condition.
- the pediatric patients are preterm, near term or term newborns, babies, toddlers, little children, or the like.
- the ventilation apparatus provides a non-invasive positive pressure ventilation (NPPV) to the pediatric patient.

Preferred embodiments of a pediatric nasal mask according to the present disclosure are shown in the enclosed Figures, among which :
- Figures 1 and 2 represent front and rear views of an embodiment of a nasal mask according to the present disclosure that is designed for infants of less than 3 year old.
- Figures 3 and 4 represent rear and front views of an embodiment of the one-piece assembly formed by the tubular element and the support element of the nasal mask of Fig. 1.
- Figure 5 represents a cross-sectional view (seen from above) of the nasal mask of Fig. 1.
- Figure 6 represents a sagittal sectional view (seen from a side) of the nasal mask of Fig. 1.

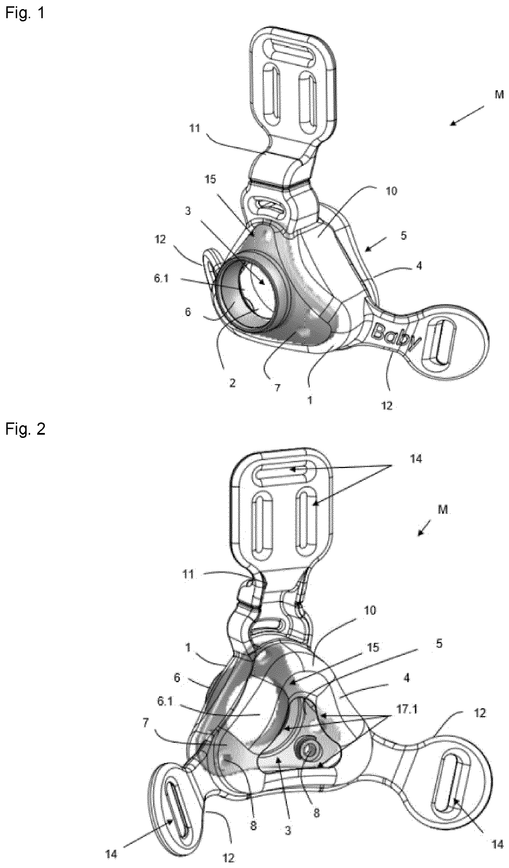

The present disclosure proposes a respiratory pediatric nasal mask M, designed and configured for being used in the pediatric field, especially designed for little infants under 3 years and/or of less than 12 kg, as illustrated in Figures 1-6 that illustrate an embodiment of a mask M according to the present disclosure.

As shown in Fig. 1-6, a pediatric nasal mask M according to the present disclosure that is configured for pediatric use comprises a one-piece hollow molded structure 10 made of a resilient soft material, preferably silicone or the like. The molded structure 10 is entirely flexible, i.e. supple.

The one-piece hollow molded structure 10 comprising a front body 1, also called mask body or front part, a rear cushion 4, also called rear part, and an inner chamber 3, i.e. an inner volume for the gas.

As shown, the front body 1 of the one-piece molded structure 10 presents a generally triangular tridimensional shape, in particular its front panel 13.

The front body 1 and the rear cushion 4 internally delimit the inner chamber 3. There are made one-piece by injection molding or the like. They cannot be detached one from the other.

The front body 1 that forms a front part or the mask M comprises an inlet orifice 2 that opens in the inner chamber 3. In other words, the inlet orifice 2 traverses the front panel 13, i.e. the front wall, of the front body 1.

As shown in Fig. 1-2, the inlet orifice 2 traversing the front panel 13 of the front body 1 of the mask M, is arranged at the center of the front panel 13. Said front panel 13 is preferably also curved, in particular the curvature of the front panel 13 approximatively equals the curvature of the support element 7 bearing the tubular element 6.

Further, the cushion 4 comprises a rear aperture 5 that is adapted, i.e. designed and/or dimensioned, for receiving at least part of the infant's nose, when the infant wears the mask M. The rear aperture 5 is also in fluid communication with the inner chamber 3 of the mask M.

Further, a tubular element 6 made of a rigid polymer, i.e. plastic or the like, is arranged in the inlet orifice 2 of the front body 1. The tubular element 6 comprises an inner passage 6.1, i.e. a lumen.

For instance, the tubular element 6 can have an inner diameter of less than 20 mm, preferably of between 12 and 19 mm, more preferably of between 14 and 17 mm.

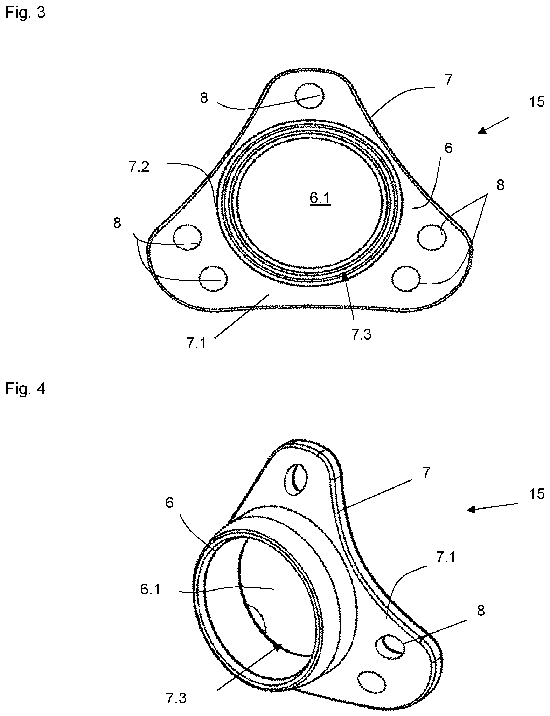

As shown in Fig. 3-4, the tubular element 6 is arranged on a support element 7, and the one-piece molded structure 10 is over-molded over said support element 7.

In the embodiment of Fig. 3-4, the support element 7 comprises a plate structure. The plate structure is curved and has a generally-triangular shape. The vertices of the curved triangular-shaped plate structure are rounded.

For instance, the triangular-shaped support element 7 can have a height of between 25 and 35 mm and a base of between 30 and 40 mm in length, and a thickness of between 1.5 and 3 mm.

Further, the support element 7, i.e. the plate structure, comprises several traversing holes 8 that are filled with resilient soft material, such as silicone or the like, during the over-molding process thereby firmly securing the support element 7, namely the plate structure, to the one-piece molded structure 10.

The tubular element 6 and the support element 7 form a one-piece assembly 15, preferably a rigid or semi-rigid assembly. It can be manufactured by injection molding or any suitable manufacturing process. It is preferably made of plastic material, such as polyamide (PA), polycarbonate (PC) and polybutylene terephthalate (PBT), or any other plastic/polymer material.

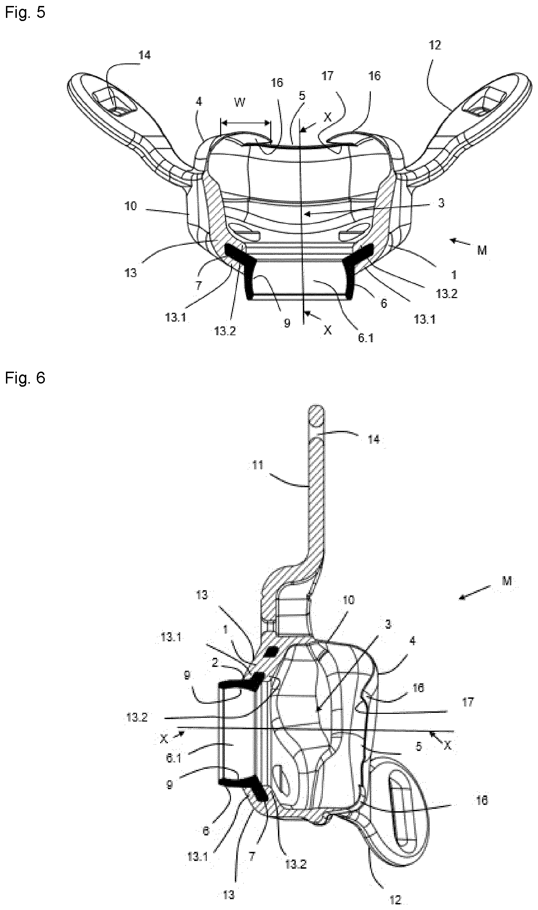

Furthermore, as shown in Fig. 5-6, as the one-piece molded structure 10 is over-molded over said support element 7, the front panel 13 is split in two sub-walls, namely an outer wall 13.1 and an inner wall 13.2. The support element 7, especially the curved plate structure, is sandwiched and firmly secured between the outer and inner walls 13.1, 13.2 of the front panel 13.

The support element 7 and the tubular element 6 are preferably made of one-piece of a rigid polymer, i.e. a plastic material. The tubular element 6 protrudes on a first surface 7.1 of the support element 7, i.e. its front surface.

The support element 7 is traversed by an opening 7.3 that is fluid communication with the inner passage 6.1 of the tubular element 6. In other words, the opening 7.3 extends from the second surface 7.2, i.e. its rear surface, to the first surface 7.1 of the support element 7 through the thickness, i.e. wall, of the support element 7, namely the curved plate structure.

The opening 7.3 and the inner passage 6.1 of the tubular element 6 are coaxially arranged (XX). The inner diameter of the opening 7.3 equals the inner diameter of the inner passage 6.1 of the tubular element 6.

As better shown in Fig. 5-6, the tubular element 6 comprises an inner wall 9 forming a ball-socket structure.

Preferably, at least a part of the inner wall 9 of the tubular element 6 has a semi-spherical shape, i.e. partially spherical. In other words, at least a part of the inner wall 9 of the tubular element 6 is configured to lodge a ball-end of a tubular connector (not shown), i.e. a connector with a (partially) spherical head, that is inserted and rotatably maintained in the ball-socket structure of the tubular element 6, preferably, an elbow connector.

When lodged in the tubular element 6, the ball-end of the tubular connector cooperates with the inner wall 9 of the tubular element 6 so that the connector is retained therein, while being rotatable.

Indeed, in use, a respiratory gas, such as air under pressure, is introduced in the mask M, namely in the inner chamber 3 of the one-piece hollow molded structure 10, by means of a flexible hose equipped with a ball-end connector that is firmly rotatably maintained in the tubular element 6. The flexible hose is fed with pressurized air or any other suitable respiratory gas by a gas delivery device, such as a medical ventilator or the like. Typically, the gas is provide at a pressure of between about 4 and 24 cmH₂O.

The mask body 1 receives at least a part of the patient's nose, when said patient introduces his/her nose through the rear aperture 5, i.e. enlarged opening for the nose, located at the rear of the mask M, for breathing of the gas contained in the inner chamber 3 of the one-piece hollow molded structure 10.

In other words, the cushion 4 comprises a rear aperture 5 adapted for receiving at least part of the infant's nose, when the infant wears the mask, said rear aperture 5 being in fluid communication with the inner chamber 3 of the one-piece hollow molded structure 10. The cushion 4 further ensures gas tightness and comfort of use for the infant. Additional details are given below.

Further, a pediatric nasals mask M according to the disclosure, as shown in Figures 1-6, also comprises a holding arm, also called upper arm, 11 forming an upper or frontal support that is integral with the one-piece hollow molded structure 10 and projects upwardly from said one-piece hollow molded structure 10 and, further, two lateral arms 12 that are also integrally arranged on the one-piece hollow molded structure 10 and that project laterally, i.e. on each opposite lateral sides (right and left sides) from said one-piece hollow molded structure 10.

Those lateral arms 12 may also constitute right and left cheek supports, when the mask is donned by a patient, i.e. an infant to be treated. They are also used for connecting a headgear thereto, as explained below.

As the mask M should provide efficient gas tightness (seal) and/or good comfort for a pediatric patient, i.e. an infant of under 3 years and/or of less than 12 kg, such as a newborn, a baby, a toddler, a child or the like, the mask of the present disclosure, is preferably designed for being very light.

In this scope, the weight of the one-piece hollow molded structure 10 including the one-piece assembly 15 comprising the tubular element 6 and the support element 7, the holding arm 11 and the two lateral arms 12 that form the main elements of the mask M (i.e. without tubular connector and headgear), does not exceed 50 grams, preferably it is less than 40 g, more preferably less than 30 g, even more preferably of between 12 and 20 g, i.e. the lighter the better.

Further, the pediatric mask M, especially the cushion 4, should match the morphology of the nasal regions of the infant to be treated and be comfortable when donned by the infant, while ensuring gas tightness.

In this aim, the one-piece hollow molded structure 10, the holding arm 11 and the two lateral arms 12 are made of a single piece of a resilient soft material, preferably silicone. The thus obtained, mask structure made of resilient soft material is entirely flexible and can hence be adapted to a great variety of facial morphologies of the nasal region of the younger infants, such as children, babies, newborns or toddlers, of up to 3 years and/or of less than 12 kg, typically of between 3 and 12 kg.

Using a soft material also avoids the risks that some pediatric patients encounter facial deformations or could be harmed due to an excessive rigidity of the mask M or any element of it.

The soft resilient rear cushion 4, that comes into contact with the infant's face, during use of the mask, and receives the nose of the infant in its central rear aperture 5, comprises a soft flexible membrane 16 forming a skirt delimits the rear aperture 5. Said soft flexible membrane 16 surrounds the central rear aperture 5 and is molded in one piece with the rest of the cushion 4 so as to integrally form part of the one-piece hollow molded structure 10.

The thickness of said soft flexible membrane 16 is preferably equal, i.e. the same, all around the rear aperture 5, namely in the different regions of the membrane 16 surrounding the rear aperture 5 as shown in Fig. 6. For instance, it thickness might be of less than 1.5 mm, preferably of less than 1 mm, for instance of about 0.5 mm, thereby providing a great flexibility that allows it to better match the contours of nasal regions of the infant, when the mask M is donned. In particular, the soft flexible membrane 16 is configured to flex or bent while matching the contours of nasal regions of the infant.

In the embodiments of Fig. 1-6, a unique membrane 16 is provided as using several membranes is not necessary. However, several superimposed membranes or the like, such as two superimposed membranes, might be used in other embodiments.

Further, the soft flexible membrane 16 comprises a peripheral rim 17 or border delimiting the rear aperture 4 as illustrated in Fig. 5-6. The regions of the flexible membrane 16 comprising the peripheral rim 17 can be flat, i.e. included in a plane, or slightly incurved toward the inner volume of the cushion 4, i.e. the inner chamber 3.

As shown in Fig. 2, the peripheral rim 17 of the rear aperture 5 of the cushion 4, in particular of the flexible membrane 16, has generally-triangular shape with side borders 17.1 having a curved shape oriented toward the center of the rear aperture 5.

Preferably, the soft flexible membrane 16 has a width W of between about 9 and 15 mm, as shown in Fig. 6. Said width is preferably equal/constant all around the rear aperture 5, i.e. in the regions of the soft flexible membrane 16 that are in contact with the nasal regions of the infant, when the mask is donned.

As already mentioned, the pediatric mask M, especially the one-piece hollow molded structure 10, has a generally-triangular tridimensional general shape as illustrated in Fig. 1-2. As the mask is to be donned by young pediatric patients, it should not be too thick or too big.

For instance, the one-piece hollow molded structure 10 can have the following dimensions for fitting to the nasal region of an infant under 3 years (i.e. newborn, baby, toddler, little child or the like) and/or weighting less than 12 kg, preferably of between about 3 kg and 12 kg, namely a height of about 87 mm and a width of about 83 mm.

As the mask M of the present disclosure is a nasal mask, the cushion 4, including the flexible membrane 16, can comprise an upper nasal bridge region, a lower region and two lateral regions (i.e. left and right regions) connecting the nasal bridge and lower regions. When the mask is donned by the infant, the upper nasal bridge region is roughly in contact with regions of the nasal bridge of the infant, the lower region is roughly in contact with the area between the nose and the upper lip of the infant, i.e. regions located under the nose, and the lateral opposite regions are roughly in contact with the flange regions of the nose of the infant.

As shown in Fig. 1 and 2, the mask M, especially the one-piece hollow molded structure 10, has a generally triangular tridimensional shape for better matching or corresponding to the general structure of a nose of a pediatric patient, while he holding arm 11 projects upwardly from one of the three vertices or corners (i.e. angle regions) of the triangular structure, whereas the two lateral arms 12 project laterally from the two other vertices of the mask M, especially of the one-piece hollow molded structure 10.

As shown in Figures 1-2 and 5-6, the holding arm 11 and the two lateral arms 12 comprise slots 14 or similar traversing openings of any shapes, located at their terminal ends, for receiving and fixing straps of a headgear thereto (not shown), that are required for maintaining the mask M in a desired position on the head of the patient during its use.

A nasal respiratory mask M of the present disclosure can be used in a method for treatment of a respiratory disorder or condition affecting pediatric patients, especially infants under 3 years and/or of less than 12 kg, such as preterm, near term or term newborns, babies, toddlers, little children, or the like, in non-invasive positive pressure ventilation (NPPV) or any other respiratory disease affecting said pediatric patients.

## Claims

1. Pediatric nasal mask (M) comprising :
- a one-piece hollow molded structure (10) made of a resilient soft material, said one-piece hollow molded structure (10) comprising a front body (1), a rear cushion (4) and an inner chamber (3), wherein :
. the front body (1) comprises an inlet orifice (2) opening in the inner chamber (3), and
. the cushion (4) comprises a rear aperture (5) adapted for receiving at least part of the infant's nose, when the infant wears the mask, said rear aperture (5) being in fluid communication with the inner chamber (3),
- and a tubular element (6) made of a rigid polymer arranged in the inlet orifice (2) of the front body (1),
**characterized in that** the tubular element (6) is arranged on a support element (7), the support element (7) being a plate structure having a curved triangular shape with rounded vertices, and the one-piece molded structure (10) is over-molded over said support element (7).

2. Mask according to Claim 1, **characterized in that** the triangular-shaped plate structure has a height of between 25 mm and 35 mm and a base of between 30 mm and 40 mm.

3. Mask according to Claim 2, **characterized in that** the triangular-shaped plate structure has a wall thickness of between 1.5 mm and 3 mm.

4. Mask according to any one of Claims 1 to 3, **characterized in that** the tubular element (6) is arranged at the center of the triangular-shaped plate structure forming the support element (7).

5. Mask according to any one of Claims 2 to 4, **characterized in that** the support element (7) comprises several traversing holes (8).

6. Mask according to any one of the preceding Claims, **characterized in that** the support element (7) and the tubular element (6) are made of one-piece of a rigid polymer, i.e. a plastic material.

7. Mask according to Claim 1, **characterized in that** the one-piece molded structure (10) further comprises a holding arm (11) projecting upwardly and two lateral arms (12) arranged on each side and projecting laterally, the front body (1), the holding arm (11), the rear cushion (4) and the two lateral arms (12) being molded in one piece and made of the resilient soft material.

8. Mask according to any one of Claims 1 or 7, **characterized in that** the resilient soft material comprises silicone.

9. Mask according to Claim 1, **characterized in that** the tubular element (6) comprises an inner wall (9) forming a ball-socket structure.

10. Mask according to Claim 9, **characterized in that** it comprises a tubular connector comprising a ball-end rotatably inserted into the ball-socket structure of the tubular element (6).

11. Mask according to any one of the preceding Claims, **characterized in that** the cushion (4) comprises at least one flexible membrane (16) surrounding the central aperture (5).

12. Mask according to Claim 7, **characterized in that** a headgear is attached to the holding arm (11) and the two lateral arms (12).

13. Mask according to any one of the preceding Claims, **characterized in that** it is a nasal mask dimensioned for fitting to the nasal region of an infant, newborn, baby, toddler or child under 3 years and/or of less than 12 kg.

14. Ventilation assembly comprising a gas delivery device and a pediatric mask (M) according to any one of the preceding Claims, said gas delivery device being fluidly connected to the pediatric mask (M) by means of a flexible hose for providing a respiratory gas to the pediatric mask (M) having a pressure of between 4 and 24 cmH20.

15. Ventilation assembly according to Claim 14, **characterized in that** the flexible hose comprises a tubular connector comprising a ball-end, said ball-end being rotatably coupled to the ball-socket structure of the tubular element (6) of the pediatric mask (M).

## Patentansprüche

1. Pädiatrische Nasenmaske (M), umfassend:
- einer einteiligen, hohlgeformten Struktur (10) aus einem elastischen, weichen Material, wobei die einteilige, hohlgeformte Struktur (10) einen vorderen Körper (1), ein hinteres Polster (4) und eine Innenkammer (3) umfasst, wobei:
. der vordere Körper (1) eine Einlassöffnung (2) aufweist, die in die Innenkammer (3) mündet, und
. das Polster (4) eine hintere Öffnung (5) aufweist, die dazu ausgelegt ist, zumindest einen Teil der Nase des Säuglings aufzunehmen, wenn dieser die Maske trägt, wobei die hintere Öffnung (5) in Fluidverbindung mit der Innenkammer (3) steht,
- sowie ein röhrenförmiges Element (6) aus einem starren Polymer, das in der Einlassöffnung (2) des vorderen Körpers (1) angeordnet ist,
**dadurch gekennzeichnet, dass** das röhrenförmige Element (6) auf einem Stützelement (7) angeordnet ist, wobei das Stützelement (7) eine Plattenstruktur mit einer gekrümmten dreieckigen Form mit abgerundeten Eckpunkten ist, und die einteilige Formstruktur (10) über das Stützelement (7) hin überspritzt ist.

2. Maske nach Anspruch 1, **dadurch gekennzeichnet, dass** die dreieckförmige Plattenstruktur eine Höhe zwischen 25 mm und 35 mm und eine Grundseite zwischen 30 mm und 40 mm aufweist.

3. Maske nach Anspruch 2, **dadurch gekennzeichnet, dass** die dreieckförmige Plattenkonstruktion eine Wandstärke zwischen 1,5 mm und 3 mm aufweist.

4. Maske nach einem der Ansprüche 1 bis 3, **dadurch gekennzeichnet, dass** das rohrförmige Element (6) in der Mitte der das Stützelement (7) bildenden dreieckförmigen Plattenstruktur angeordnet ist.

5. Maske nach einem der Ansprüche 2 bis 4, **dadurch gekennzeichnet, dass** das Stützelement (7) mehrere Durchgangslöcher (8) aufweist.

6. Maske nach einem der vorstehenden Ansprüche, **dadurch gekennzeichnet, dass** das Stützelement (7) und das rohrförmige Element (6) aus einem einteiligen Stück eines starren Polymers, d. h. eines Kunststoffmaterials, bestehen.

7. Maske nach Anspruch 1, **dadurch gekennzeichnet, dass** die einteilige Formstruktur (10) ferner einen nach oben ragenden Haltearm (11) und zwei seitlich angeordnete, seitlich ragende Seitenarme (12) umfasst, wobei der vordere Körper (1), der Haltearm (11), das hintere Polster (4) und die beiden seitlichen Arme (12) einstückig geformt sind und aus dem elastischen, weichen Material bestehen.

8. Maske nach einem der Ansprüche 1 oder 7, **dadurch gekennzeichnet, dass** das elastische, weiche Material Silikon umfasst.

9. Maske nach Anspruch 1, **dadurch gekennzeichnet, dass** das röhrenförmige Element (6) eine Innenwand (9) aufweist, die eine Kugelgelenkstruktur bildet.

10. Maske nach Anspruch 9, **dadurch gekennzeichnet, dass** sie ein röhrenförmiges Verbindungselement umfasst, das ein Kugelende aufweist, das drehbar in die Kugel-Pfannen-Struktur des röhrenförmigen Elements (6) eingesetzt ist.

11. Maske nach einem der vorstehenden Ansprüche, **dadurch gekennzeichnet, dass** das Polster (4) mindestens eine flexible Membran (16) umfasst, die die zentrale Öffnung (5) umgibt.

12. Maske nach Anspruch 7, **dadurch gekennzeichnet, dass** am Haltearm (11) und an den beiden seitlichen Armen (12) ein Kopfband befestigt ist.

13. Maske nach einem der vorstehenden Ansprüche, **dadurch gekennzeichnet, dass** es sich um eine Nasenmaske handelt, die für die Anpassung an den Nasenbereich eines Säuglings, Neugeborenen, Babys, Kleinkindes oder Kindes unter 3 Jahren und/oder mit einem Gewicht von weniger als 12 kg ausgelegt ist.

14. Beatmungsvorrichtung, umfassend eine Gaszufuhrvorrichtung und eine pädiatrische Maske (M) gemäß einem der vorstehenden Ansprüche, wobei die Gaszufuhrvorrichtung mittels eines flexiblen Schlauchs zur der pädiatrischen Maske (M) fluidisch verbunden ist, um der pädiatrischen Maske (M) ein Atemgas mit einem Druck zwischen 4 und 24 cmH□O zuzuführen.

15. Beatmungsvorrichtung nach Anspruch 14, **dadurch gekennzeichnet, dass** der flexible Schlauch ein rohrförmiges Verbindungsstück mit einem Kugelende umfasst, wobei das Kugelende drehbar mit der Kugel-Pfannen-Struktur des rohrförmigen Elements (6) der Kindermaske (M) gekoppelt ist.

## Revendications

1. Masque nasal pédiatrique (M) comprenant :
- une structure moulée creuse d'une seule pièce (10) réalisée en un matériau souple et élastique, ladite structure moulée creuse d'une seule pièce (10) comprenant un corps avant (1), un coussin arrière (4) et une chambre interne (3), dans laquelle :
. le corps avant (1) comporte un orifice d'entrée (2) débouchant dans la chambre interne (3), et
. le coussin (4) comporte une ouverture arrière (5) adaptée pour recevoir au moins une partie du nez du nourrisson lorsque celui-ci porte le masque, ladite ouverture arrière (5) étant en communication fluidique avec la chambre interne (3),
- et un élément tubulaire (6) en polymère rigide disposé dans l'orifice d'entrée (2) du corps avant (1),
**caractérisé en ce que** l'élément tubulaire (6) est disposé sur un élément de support (7), l'élément de support (7) étant une structure en plaque présentant une forme triangulaire incurvée avec des sommets arrondis, et la structure moulée d'un seul tenant (10) est surmoulée sur ledit élément de support (7).

2. Masque selon la revendication 1, **caractérisé en ce que** la structure en plaque de forme triangulaire présente une hauteur comprise entre 25 mm et 35 mm et une base comprise entre 30 mm et 40 mm.

3. Masque selon la revendication 2, **caractérisé en ce que** la structure en plaque de forme triangulaire présente une épaisseur de paroi comprise entre 1,5 mm et 3 mm.

4. Masque selon l'une quelconque des revendications 1 à 3, **caractérisé en ce que** l'élément tubulaire (6) est disposé au centre de la structure en plaque de forme triangulaire formant l'élément de support (7).

5. Masque selon l'une quelconque des revendications 2 à 4, **caractérisé en ce que** l'élément de support (7) comprend plusieurs trous traversants (8).

6. Masque selon l'une quelconque des revendications précédentes, **caractérisé en ce que** l'élément de support (7) et l'élément tubulaire (6) sont réalisés d'une seule pièce en un polymère rigide, c'est-à-dire en une matière plastique.

7. Masque selon la revendication 1, **caractérisé en ce que** la structure moulée d'un seul tenant (10) comprend en outre un bras de maintien (11) s'étendant vers le haut et deux bras latéraux (12) disposés de chaque côté et s'étendant latéralement, le corps avant (1), le bras de maintien (11), le coussin arrière (4) et les deux bras latéraux (12) étant moulés d'un seul tenant et réalisés en matériau souple et élastique.

8. Masque selon l'une quelconque des revendications 1 ou 7, **caractérisé en ce que** le matériau souple et élastique comprend de la silicone.

9. Masque selon la revendication 1, **caractérisé en ce que** l'élément tubulaire (6) comprend une paroi interne (9) formant une structure à rotule.

10. Masque selon la revendication 9, **caractérisé en ce qu'**il comprend un connecteur tubulaire comportant une extrémité sphérique insérée de manière rotative dans la structure à rotule de l'élément tubulaire (6).

11. Masque selon l'une quelconque des revendications précédentes, **caractérisé en ce que** le coussin (4) comprend au moins une membrane flexible (16) entourant l'ouverture centrale (5).

12. Masque selon la revendication 7, **caractérisé en ce qu'**un harnais est fixé au bras de maintien (11) et aux deux bras latéraux (12).

13. Masque selon l'une quelconque des revendications précédentes, **caractérisé en ce qu'**il s'agit d'un masque nasal dimensionné pour s'adapter à la région nasale d'un nourrisson, d'un nouveau-né, d'un bébé, d'un enfant en bas âge ou d'un enfant de moins de 3 ans et/ou pesant moins de 12 kg.

14. Ensemble de ventilation comprenant un dispositif de délivrance de gaz et un masque pédiatrique (M) selon l'une quelconque des revendications précédentes, ledit dispositif de délivrance de gaz est relié de manière fluidique au masque pédiatrique (M) au moyen d'un tuyau flexible fournissant un gaz respiratoire au masque pédiatrique (M) à une pression comprise entre 4 et 24 cmH₂O.

15. Ensemble de ventilation selon la revendication 14, **caractérisé en ce que** le tuyau flexible comprend un raccord tubulaire comportant une extrémité sphérique, ladite extrémité sphérique étant couplée de manière rotative à la structure à rotule de l'élément tubulaire (6) du masque pédiatrique (M).
